(19) **Europäisches Patentamt / European Patent Office / Office européen des brevets**

(11) **EP 4 635 504 A1**

(12) **EUROPEAN PATENT APPLICATION**
published in accordance with Art. 153(4) EPC

(43) Date of publication:
22.10.2025 Bulletin 2025/43

(21) Application number: 23903506.6

(22) Date of filing: 12.12.2023

(51) International Patent Classification (IPC):
A61K 35/15 (2025.01)  A61K 35/14 (2015.01)
A61K 35/28 (2015.01)  A61K 35/51 (2015.01)
A61P 1/04 (2006.01)  A61P 1/16 (2006.01)
A61P 9/00 (2006.01)  A61P 9/04 (2006.01)
A61P 9/10 (2006.01)  A61P 17/02 (2006.01)
A61P 19/02 (2006.01)  A61P 25/00 (2006.01)
A61P 25/14 (2006.01)  A61P 25/16 (2006.01)
A61P 25/28 (2006.01)  A61P 29/00 (2006.01)
A61P 35/00 (2006.01)  A61P 37/02 (2006.01)
A61P 37/06 (2006.01)  A61P 43/00 (2006.01)

(52) Cooperative Patent Classification (CPC):
A61K 35/14; A61K 35/15; A61K 35/28;
A61K 35/51; A61P 1/04; A61P 1/16; A61P 9/00;
A61P 9/04; A61P 9/10; A61P 17/02; A61P 19/02;
A61P 25/00; A61P 25/14; A61P 25/16; A61P 25/28;
(Cont.)

(86) International application number:
PCT/JP2023/044423

(87) International publication number:
WO 2024/128222 (20.06.2024 Gazette 2024/25)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 12.12.2022 JP 2022197874

(71) Applicant: Juntendo Educational Foundation
Tokyo 113-8421 (JP)

(72) Inventors:
• TANAKA, Rica
Tokyo 113-8421 (JP)
• FURUKAWA, Satomi
Tokyo 113-8421 (JP)

(74) Representative: Mewburn Ellis LLP
Aurora Building
Counterslip
Bristol BS1 6BX (GB)

(54) **COMPOSITION AND USE THEREOF**

(57) The present invention relates to a composition and a method. The composition of the present invention is a composition for a treatment containing a mesenchymal stem cell and not containing a mononuclear cell fraction, and is produced by co-culturing ae mesenchymal stem cell with an in vitro amplified and cultured mononuclear cell fraction and separating the in vitro amplified and cultured mononuclear cell fraction prior to the treatment.

Fig. 1

(Cont. next page)

(52) Cooperative Patent Classification (CPC): (Cont.)
**A61P 29/00; A61P 35/00; A61P 37/02;**
**A61P 37/06; A61P 43/00; C12N 5/06**

## Description

TECHNICAL FIELD

The present invention relates to a composition and use thereof.

BACKGROUND ART

In recent years, treatments using mesenchymal stem cells have been studied in many diseases. The mesenchymal stem cells are somatic stem cells derived from mesodermal tissue (mesenchyme), and have potency to differentiate into cells belonging to the mesenchymal cell. Depending on the tissue from which the mesenchymal stem cells are collected, they are called adipose-derived mesenchymal stem cells, bone marrow-derived mesenchymal stem cells, and the like. Mesenchymal stem cells are known to have pluripotency to differentiate into various somatic cells, such as bone, blood vessel, and cardiac muscle cells, and also secrete cytokines and have an immunosuppressive effect, an anti-inflammatory effect, and an angiogenic effect. In addition, mesenchymal stem cells can be grafted not only autologously but also allogeneically, due to their high immunotolerance capability.

Due to these characteristics, mesenchymal stem cells are pluripotent stem cells with great potential in regenerative medicine. Up to the present time, numerous clinical studies thereon have been conducted for various diseases, and they are expected to be used to treat a wide variety of diseases. However, at the present time, a treatment with mesenchymal stem cells alone has not achieved a satisfactory effect in reality.

Breast cancer is cancer with the highest morbidity in women, and its morbidity tends to increase year by year. Approximately 40% of patients with breast cancer require mastectomy (2017). The mastectomy brings great suffering, especially mentally. Fat grafting is performed in the field of soft tissue reconstruction, such as breast reconstruction after breast cancer surgery, for the purpose of increasing the volume and restoring the contour. This technique uses abundantly available adipose tissue as an autologous filler, which can achieve a natural feel, and is an inexpensive treatment with a low infection rate. Thus, adipose tissue is superior to exogenous materials. However, during the process of collecting fat by liposuction, adipocytes are cut off from original blood supply. As a result, the graft after its injection undergoes temporary ischemia, which causes partial necrosis and graft volume loss (NPL 1). Fat grafting and its long-term clinical application have been drawing increasing attention (NPL 2). However, a satisfactory solution to such volume loss due to ischemia still remains to be developed. In order for fat grafting to achieve satisfactory results, a secondary treatment is often required. It is demanded to develop a fat grafting technique which achieves higher fat regeneration and angiogenesis capabilities.

As a cell-based therapy for enhancing fat grafting, adipose-derived mesenchymal stem cells (ASCs) have been studied (NPL 20). A therapy in which ASCs are supplemented when adipocytes are grafted (ASC-supplemented fat grafting) is promising, but requires further development in order to obtain consistent clinical results (NPLs 21 and 22). ASCs have been observed to release proangiogenic cytokines (NPL 23) and differentiate into vascular endothelium in vitro (NPL 24). However, on the other hand, in vivo experiments using ASCs suggest that the effect on vascularization is mainly based on paracrine (NPL 25), or that ASCs enhance fat grafting via a different, non-angiogenic mechanism (NPL 26). However, as an issue, use of ASCs alone is not sufficient for the adipose tissue to be engrafted by angiogenesis. Another issue is also caused by fibrosis. If such issues are improved, supplementation of ASCs is expected to have even higher clinical effects in the fat grafting for the purpose of breast reconstruction or the like.

Furthermore, "ischemic diseases" are diseases caused by a decrease in a blood volume, which reduces a blood flow in tissue, resulting in a histological damage, such as cellular degeneration, atrophy, and fibrosis. In particular, when "arteriosclerosis obliterans", a condition in which the arteries supplying blood to the legs become constricted or obstructed, increases in severity, it can lead to severe limb ischemia, causing symptoms such as pain and intractable ulcers. In the worst case scenario, it may require amputation of a lower limb. For the treatment of ischemic diseases, a treatment with mesenchymal stem cells has also been considered, but has not yet achieved sufficient effects. Furthermore, since fibrosis is observed in tissue after mesenchymal stem cells are grafted, it is possible that a histological damage due to fibrosis may remain. If this issue is improved, satisfactory clinical effects on limb ischemia are expected.

The present inventors revealed in WO2019/146131 (PTL 3) that a composition containing an in vitro amplified and cultured mononuclear cell fraction has an effect to increase an effect of mesenchymal stem cells. Specifically, it was found that supplementing an in vitro amplified and cultured mononuclear cell fraction to a treatment using mesenchymal stem cells safely and effectively increased effects of the treatment by stimulating vascularization and exerting an anti-inflammatory effect and an anti-immune effect, using two models: fat grafting and limb ischemia. These effects are observed not only in one disease but in a plurality of diseases for which mesenchymal stem cells exert effects. This result supports that an in vitro amplified and cultured mononuclear cell fraction increases the characteristics of mesenchymal stem cells and makes it possible to improve a treatment of a disease for which a conventional treatment with mesenchymal stem cells has not achieved sufficient effects.

These effects to increase effects of the mesenchymal stem cells were observed when the mesenchymal stem cells were co-grafted with an in vitro amplified and cultured mononuclear cell fraction which had been co-cultured therewith.

WO2019/146131 describes a composition for increasing an effect of a treatment using a mesenchymal stem cell, which contains an in vitro amplified and cultured mononuclear cell fraction, and a method for increasing an effect of a treatment using a mesenchymal stem cell, which includes using a composition containing an in vitro amplified and cultured mononuclear cell fraction together with the mesenchymal stem cell or a composition containing the mesenchymal cell.

CITATION LIST

PATENT LITERATURE

PTL 1: WO2006/090882
PTL 2: WO2014/0561154
PTL 3: WO2019/146131
PTL 4: WO2021/131261

NON PATENT LITERATURE

NPL 1: Plast Reconstr Surg. 2013 Feb; 131(2): 185-191
NPL 2: Langenbecks Arch Klin Chir Ver Dtsch Z Chir. 1893; 22: 66-69
NPL 3: Otolaryngol Head Neck Surg. 1990 Apr; 102(4): 314-321
NPL 4: Ann Plast Surg. 2005 Jul; 55(1): 63-68
NPL 5: Exp Biol Med (Maywood). 2005 Nov; 230(10): 742-748
NPL 6: Dermatol Surg. 2006 Dec; 32(12): 1437-1443
NPL 7: Plast Reconstr Surg Glob Open. 2013 Oct 7; 1(6): e40
NPL 8: Arch Plast Surg. 2015 Mar; 42(2): 150-158
NPL 9: Plast Reconstr Surg. 2012 May; 129(5): 1081-1092
NPL 10: Plast Reconstr Surg. 2015 Jun; 135(6): 1607-1617
NPL 11: Science. 1997 Feb 14; 275(5302): 964-967
NPL 12: Circulation. 2001 Feb 6; 103(5): 634-637
NPL 13: Nat Med. 2001 Apr; 7(4): 430-436
NPL 14: Circ Res. 2001 Jul 6; 89(1): E1-7
NPL 15: J Am Coll Cardiol. 2003 Dec 17; 42(12): 2073-2080
NPL 16: FASEB J. 2005 Jun; 19(8): 992-994
NPL 17: Plast Reconstr Surg. 2008 Jun; 121(6): 1929-1942
NPL 18: Diabetes. 2010 Aug; 59(8): 1974-1983
NPL 19: Stem Cells Transl Med. 2012 Feb; 1(2): 160-171
NPL 20: Mol Biol Cell. 2002 Dec; 13(12): 4279-4295
NPL 21: J Plast Reconstr Aesthet Surg. 2013 Nov; 66(11): 1494-1503
NPL 22: Aesthet Surg J. 2017 Jul 1; 37(suppl_3): S46-58
NPL 23: Stem Cells. 2009 Jan; 27(1): 266-274
NPL 24: Circulation. 2004 Feb 10; 109(5): 656-663
NPL 25: PLoS One. 2012; 7(9): e45621
NPL 26: Lancet. 2013 Sep 28; 382(9898): 1113-1120
NPL 27: Diabetes. 2013 Sep; 62(9): 3207-3217
NPL 28: Circ Res. 2011 Jun 24; 109(1): 20-37
NPL 29: Stem Cell Res Ther. 2013 Feb 28; 4(1): 20
NPL 30: J Am Heart Assoc. 2014 Jun 25; 3(3): e000743
NPL 31: Japanese Journal of Thrombosis and Hemostasis 2014; 25(5): 593-602

SUMMARY OF INVENTION

TECHNICAL PROBLEM

WO2019/146131 (PTL 3) describes a composition for increasing an effect of a treatment using a mesenchymal stem cell, which contains an in vitro amplified and cultured mononuclear cell fraction, and a method for increasing an effect of a treatment using a mesenchymal stem cell, which includes using a composition containing an in vitro amplified and cultured mononuclear cell fraction together with the mesenchymal stem cell or a composition containing the mesenchymal cell. The invention described in WO2019/146131 is an invention based on the premise that mesenchymal stem cells are co-cultured with an in vitro amplified and cultured mononuclear cell fraction and then both the mesenchymal stem cells and the in vitro amplified and cultured mononuclear cell fraction are co-grafted. It was considered that it would be necessary to graft also the in vitro amplified and cultured mononuclear cell fraction together with the mesenchymal stem cells.

Mesenchymal stem cells, such as adipose-derived mesenchymal stem cells (ASCs), have an immunotolerance effect and can be grafted allogeneically. Some products of human mesenchymal stem cells (MSCs) have been commercialized. On the other hand, mononuclear cell fractions cannot be grafted allogeneically. The technique described in WO2019/146131, in which mesenchymal stem cells are co-cultured with an in vitro amplified and cultured mononuclear cell fraction and then both the mesenchymal stem cells and the in vitro amplified and cultured mononuclear cell fraction are co-grafted, requires use of a mononuclear cell fraction derived from a patient (derived autologously), which was a restriction to the technique.

SOLUTION TO PROBLEM

The present inventors conducted diligent studies in order to solve the problem described above, and as a result, they found that, an effect of a treatment with mesenchymal stem cells was increased even in a case where an in vitro amplified and cultured mononuclear cell fraction and mesenchymal stem cells were co-cultured, the mesenchymal stem cells were then separated from the in vitro amplified and cultured mononuclear cell fraction, and the mesenchymal stem cells were used alone. Thus, the present inventors arrived at the present invention. The present invention non-limitingly includes the following embodiments.

[Embodiment 1] A composition for a treatment containing a mesenchymal stem cell and not containing a mononuclear cell fraction, wherein the composition is produced by:

co-culturing a mesenchymal stem cell with an in vitro amplified and cultured mononuclear cell fraction; and separating the in vitro amplified and cultured mononuclear cell fraction prior to the treatment.

[Embodiment 2] A composition containing an in vitro amplified and cultured mononuclear cell fraction for use in a method for producing a composition for a treatment containing a mesenchymal stem cell and not containing a mononuclear cell fraction, the method including:

co-culturing a mesenchymal stem cell with the in vitro amplified and cultured mononuclear cell fraction; and separating the in vitro amplified and cultured mononuclear cell fraction prior to the treatment.

[Embodiment 3] The composition according to embodiment 1 or 2, wherein the in vitro amplified and cultured mononuclear cell fraction is co-cultured with a mesenchymal stem cell for a half day to seven days.
[Embodiment 4] The composition according to any one of embodiments 1 to 3, wherein the mononuclear cell fraction is a mononuclear cell fraction derived from bone marrow, peripheral blood, or umbilical cord blood.
[Embodiment 5] The composition according to any one of embodiments 1 to 4, wherein the in vitro amplified and cultured mononuclear cell fraction is a group of cells rich in endothelial progenitor cells or anti-inflammatory cells or immunotolerance-inducing cells.
[Embodiment 6] The composition according to any one of embodiments 1 to 5, wherein the mesenchymal stem cell is an adipose-derived mesenchymal stem cell, a bone marrow-derived mesenchymal stem cell, a peripheral blood-derived mesenchymal stem cell, or an umbilical cord blood-derived mesenchymal stem cell.
[Embodiment 7] The composition according to any one of embodiments 1 to 6, wherein the treatment with the mesenchymal stem cell is a treatment in which the effect thereof is enhanced by a vascularization effect, an anti-inflammatory effect, an anti-fibrosis effect and the like by the in vitro amplified and cultured mononuclear cell fraction.
[Embodiment 8] The composition according to any one of embodiments 1 to 7, wherein the treatment with the mesenchymal stem cell is selected from the group consisting of tissue grafting, tissue regeneration, a treatment of an ischemic disease, a treatment of a neurodegenerative disease, a treatment of a heart disease, a treatment of a malignant tumor, a treatment of an inflammatory disease, and a treatment of an immune disease.
[Embodiment 9] The composition according to any one of embodiments 1 to 8, wherein the treatment with the mesenchymal stem cell is selected from the group consisting of fat grafting, bone marrow grafting, a treatment of a skeletal muscle injury, a treatment of a skin injury, tissue regeneration after prostate cancer surgery, a treatment of degenerative arthritis, a treatment of lumbar disc deformation, a treatment of a peripheral nerve injury, a treatment of limb ischemia, a treatment of ischemic ulcer, a treatment of an ischemic heart disease, a treatment of brain infarction, a treatment of amyotrophic lateral sclerosis (ALS), a treatment of Parkinson's disease, a treatment of Alzheimer's disease, a treatment of progressive supranuclear palsy, a treatment of Huntington's disease, a treatment of multiple system atrophy, a treatment of spinocerebellar degeneration, a treatment of a spinal cord injury, a treatment of cardiac failure, a treatment of endocarditis, a treatment of a heart valve disease, a treatment of pericarditis, a treatment of a congenital heart disease, a treatment of myocarditis, a treatment of myocardial infarction, a treatment of Crohn's disease, a treatment of hepatic cirrhosis, a treatment of hepatitis, a treatment of ulcerative colitis, a treatment of an inflammatory bowel disease, a treatment of a connective tissue disease, a treatment of a graft versus host disease (GVHD), and a treatment of multiple sclerosis.

[Embodiment 10] The composition according to embodiment 9, wherein the treatment with the mesenchymal stem cell is grafting, and achieves at least one of the following effects to:

(i) increase a survival rate of grafted tissue;
(ii) increase an engrafting rate of a graft;
(iii) suppress fibrosis of a grafted tissue fragment;
(iv) improve a blood flow in the grafted tissue; or
(v) reduce inflammation due to the grafting.

[Embodiment 11] The composition according to embodiment 9, wherein the treatment with the mesenchymal stem cell is a treatment of ischemia, and achieves at least one of the following effects to:

(vi) improve a blood flow;
(vii) improve a function; or
(viii) improve a histological damage.

[Embodiment 12] A method for increasing an effect of a treatment with a mesenchymal stem cell, the method including:

co-culturing an in vitro amplified and cultured mononuclear cell fraction with a mesenchymal stem cell; and
separating the mesenchymal stem cell from the in vitro amplified and cultured mononuclear cell fraction.

[Embodiment 13] The method according to embodiment 12, further including performing the treatment using the separated mesenchymal stem cell.

[Embodiment 14] A method for producing a composition for a treatment containing a mesenchymal stem cell and not containing a mononuclear cell fraction, the method including:

co-culturing a mesenchymal stem cell with an in vitro amplified and cultured mononuclear cell fraction; and
separating the in vitro amplified and cultured mononuclear cell fraction prior to the treatment.

ADVANTAGEOUS EFFECTS OF INVENTION

The composition for increasing an effect of a treatment with a mesenchymal stem cell, which contains an in vitro amplified and cultured mononuclear cell fraction according to the present invention is separated from the mesenchymal stem cell prior to the treatment with the mesenchymal stem cell. When the treatment with the mesenchymal stem cell is performed, the composition used is free of the mononuclear cell fraction and only contains the mesenchymal stem cell which can be grafted allogeneically. Thus, the mononuclear cell fraction is not limited to be derived autologously. Therefore, for both the mononuclear cell fraction and the mesenchymal stem cells, cells which are not derived from a patient but derived from a healthy human can be used. As a result, it is possible to produce a cell preparation and a pharmaceutical composition and administer them to a patient without subjecting the patient to an invasive procedure of cell collection. In addition, for preparation of the composition used in the treatment, since it can be produced and get ready before the treatment, it can be administered to the patient quickly at any time. Furthermore, by preparing the composition in advance and cryopreserving it for a long period of time, advantageous effects can be achieved, such as that it is possible to perform the treatment more quickly with reduced burden on the patient.

According to the present invention, in a treatment with a mesenchymal stem cell, one type of cell, that is, the mesenchymal stem cell, is used alone. Thus, there are less barriers to be taken into consideration in clinical application, as compared to a case in which two different types of cell (that is, the mesenchymal stem cell and the in vitro amplified and cultured mononuclear cell fraction) are used. Furthermore, when a pharmaceutical product is developed, it is easier to set cell specification and the like for a product containing one type of cell than for a product containing two types of cell.

BRIEF DESCRIPTION OF DRAWINGS

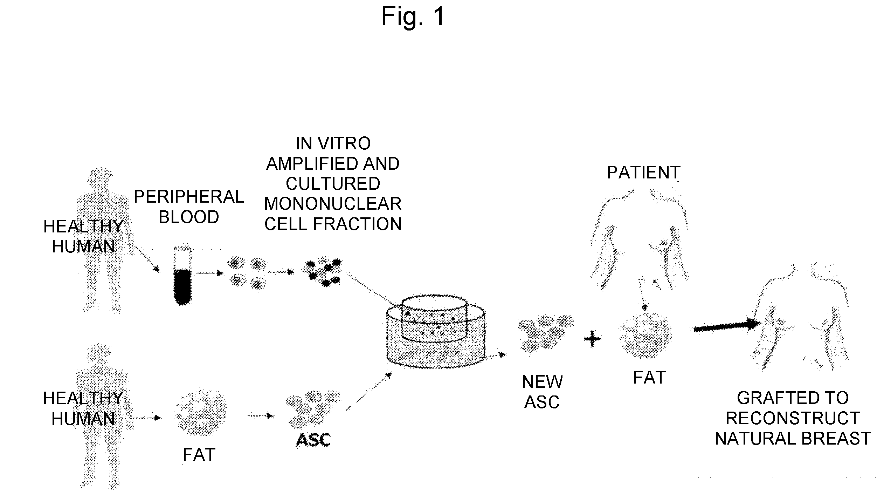

[Fig. 1] Fig. 1 is a schematic diagram illustrating embodiments of preparation of mesenchymal stem cells and fat grafting using the mesenchymal stem cells according to the present invention.

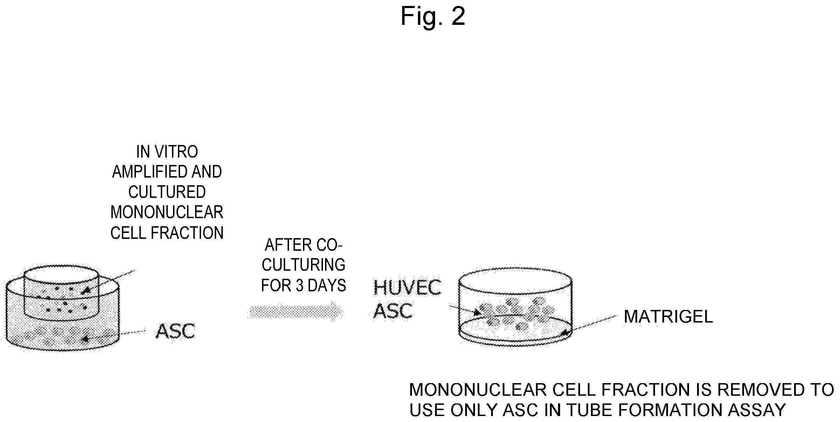

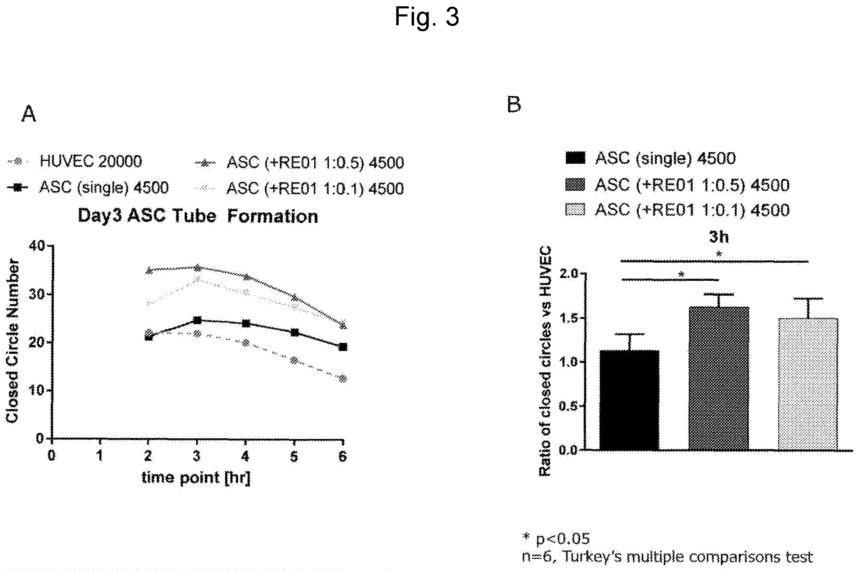

[Fig. 2] Fig. 2 is a schematic diagram illustrating one embodiment of a method in which an adipose-derived mesenchymal stem cell is prepared by co-culturing it with an in vitro amplified and cultured mononuclear cell fraction and then separating the in vitro amplified and cultured mononuclear cell fraction, and is used in a tube formation assay (results thereof are illustrated in Fig. 3).

[Fig. 3] Fig. 3 illustrates results of the tube formation assay using a composition containing mesenchymal stem cells (ASCs) according to the present invention. As ASCs, ASC 4500 cells were co-cultured with RE01 cells (in vitro amplified and cultured peripheral blood mononuclear cells) at ratios of 1:0.5 and 1:0.1, respectively, for three days, and then the RE01 cells were removed. Prepared ASC groups, ASC (+ RE01 1:0.5) 4500 group and ASC (+ RE01 1:0.1) 4500 group, were used (ASC (+ RE01 1:0.5) 4500 **(A)** and ASC (+ RE01 1:0.1) 4500 **(▼)** in Fig. 3). As controls, a group in which only HUVEC 20000 cells had been added (HUVEC20000 (●) in Fig. 3) and a group using ASC 4500 cells not co-cultured with the RE01 cells (ASC (single) 4500 (■) in Fig. 3) were used. For each group, the number of closed circles was counted. Fig. 3A illustrates changes in the number of closed circles over time. Fig. 3B illustrates a ratio of the number of closed circles of each group, to that of the HUVEC20000 group as 1.0, after three hours from the initiation of the tube formation assay.

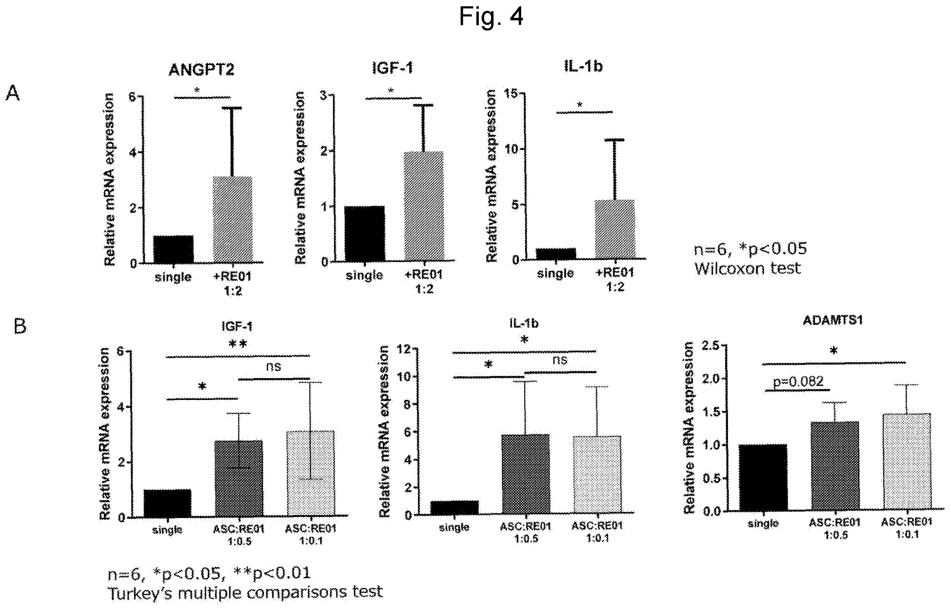

[Fig. 4] Fig. 4 illustrates results of mRNA expression analysis of ASCs which were co-cultured with RE01 cells for three days.

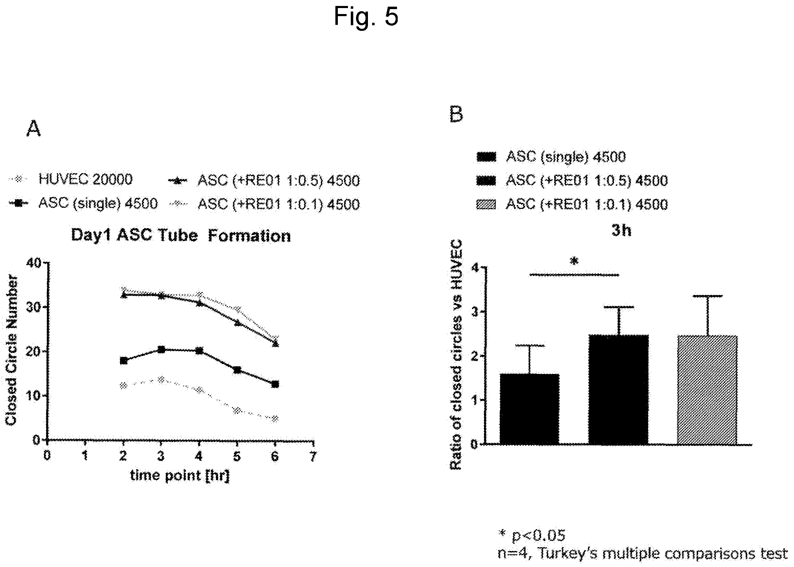

[Fig. 5] Fig. 5 illustrates results of the tube formation assay using a composition containing mesenchymal stem cells (ASCs) according to the present invention. As ASCs, ASC 4500 cells were co-cultured with RE01 cells (in vitro amplified and cultured peripheral blood mononuclear cells) at ratios of 1:0.5 and 1:0.1, respectively, for one day, and then the RE01 cells were removed. Prepared ASC groups, ASC (+ RE01 1:0.5) 4500 group and ASC (+ RE01 1:0.1) 4500 group, were used (ASC (+ RE01 1:0.5) 4500 **(A)** and ASC (+ RE01 1:0.1) 4500 **(▼)** in Fig. 5). As a control, a group in which only HUVEC 20000 cells had been added (HUVEC20000 (●) in Fig. 5) and a group using ASC 4500 cells not co-cultured with the RE01 cells (ASC (single) 4500 (■) in Fig. 5) were used. For each group, the number of closed circles was counted. Fig. 5A illustrates changes in the number of closed circles over time. Fig. 5B illustrates a ratio of the number of closed circles of each group, to that of the HUVEC20000 group as 1.0, after three hours from the initiation of the tube formation assay.

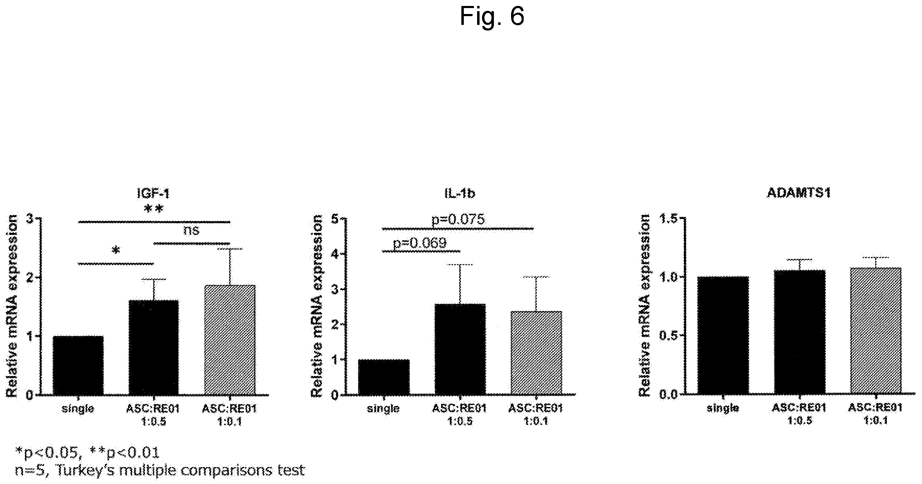

[Fig. 6] Fig. 6 illustrates results of mRNA expression analysis of ASCs which were co-cultured with RE01 cells for one day.

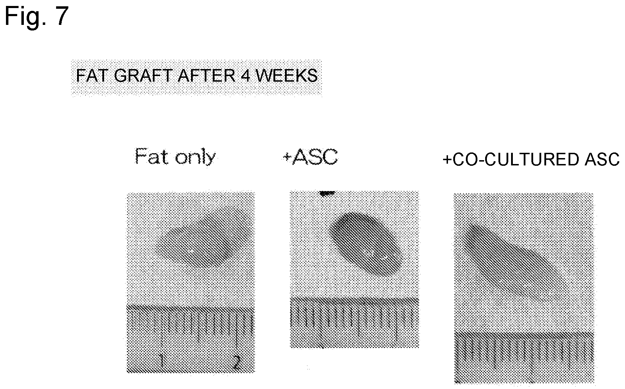

[Fig. 7] Fig. 7 illustrates grafts excised 4 weeks after grafting from nude mouse models grafted with fat to the back. "Fat only" indicates the results for a group grafted with fat alone (control), "+ ASC" indicates the results for an ASC-mixed group with conventional, not co-cultured ASCs (control), and "+ co-cultured ASC" indicates the results for a group with co-culturing with RE01 cells and subsequent separation of the RE01 (the present invention).

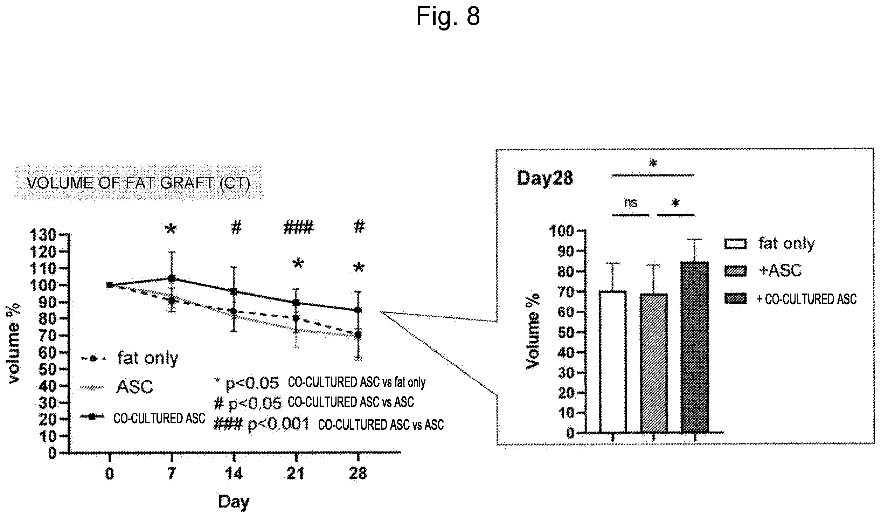

[Fig. 8] Fig. 8 illustrates changes in volume of the grafts and volume retention rates of the grafts of the nude mouse models grafted with fat to the back. "Fat only" indicates the results for a group grafted with fat alone (control), "+ ASC" indicates the results for an ASC-mixed group with conventional, not co-cultured ASCs (control), and "+ co-cultured ASC" indicates the results for a group with co-culturing with RE01 cells and subsequent separation of the RE01 (the present invention).

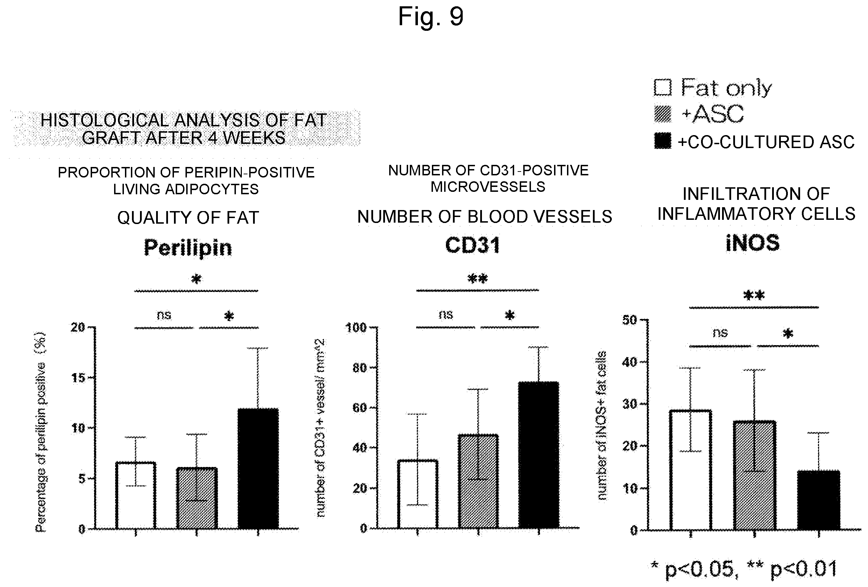

[Fig. 9] Fig. 9 illustrates results of histological analysis of the fat-grafted model mice four weeks after the grafting. The left part of Fig. 9 illustrates the proportion of the area of Perilipin antibody-positive adipocytes to the total area of the specimen (quality of fat). The center part of Fig. 9 illustrates the number of blood vessels per 1 mm$^2$. The right part of Fig. 9 illustrates the number of iNOS antibody-positive inflammatory macrophages in the entire specimen (infiltration of inflammatory cells). "Fat only" indicates the results for a group grafted with fat alone (control), "+ ASC" indicates the results for an ASC-mixed group with conventional, not co-cultured ASCs (control), and "+ co-cultured ASC" indicates the results for a group with co-culturing with RE01 cells and subsequent separation of the RE01 (the present invention).

DESCRIPTION OF EMBODIMENTS

Embodiments for implementing the present invention include the following forms.

As mentioned herein, the term "in one embodiment" means that the invention is not limited to that embodiment, that is, the embodiment is non-limiting.

1. Composition Containing In vitro amplified and cultured Mononuclear Cell Fraction

The present invention provides a composition containing an in vitro amplified and cultured mononuclear cell fraction.

In one embodiment, the composition is used in a method for producing a composition for a treatment containing a mesenchymal stem cell and not containing a mononuclear cell fraction, the method including:

co-culturing a mesenchymal stem cell with an in vitro amplified and cultured mononuclear cell fraction; and separating the in vitro amplified and cultured mononuclear cell fraction prior to the treatment.

The composition containing the in vitro amplified and cultured mononuclear cell fraction increases an effect of a treatment with the mesenchymal stem cell.

The term "mononuclear cell fraction" collectively refers to cells having a round nucleus which are contained in peripheral blood, bone marrow, umbilical cord blood, or the like obtained from an organism, and includes lymphocytes, monocytes, macrophages, endothelial progenitor cells, hematopoietic stem cells, and the like. The mononuclear cells further include CD34-positive and/or CD133-positive cells. They are obtained by collecting bone marrow, umbilical cord blood, or peripheral blood from an animal and subjecting it to a density gradient centrifugation method, for example, to extract the fraction.

In one embodiment, the mononuclear cell fraction is a mononuclear cell fraction derived from bone marrow, peripheral blood, or umbilical cord blood.

In one embodiment, the in vitro amplified and cultured mononuclear cell fraction is a group of cells rich in endothelial progenitor cells or anti-inflammatory cells or immunotolerance-inducing cells.

The term "endothelial progenitor cell (EPC)" particularly refers to CD34-positive and/or CD133-positive cells among the mononuclear cell fraction. It also includes differentiated EPC colony-forming cells. Endothelial progenitor cells are known as cells capable of differentiating into blood vessels, and are believed to be capable of repairing or supplementing a damaged blood vessel.

In one embodiment, a group of "anti-inflammatory cells or immunotolerance-inducing cells" are a group of cells rich in endothelial progenitor cells, M2 macrophages, T-lymphocyte subsets, or regulatory T-cells. These cells are believed to have an anti-inflammatory or immunotolerance-inducing function (NPL 31).

The term "in vitro amplification (Quality and Quantity) (QQ)" refers to culturing stem cells collected from an organism in vitro in a serum-free medium or a serum medium containing a factor, such as a stem cell growth factor and interleukin, for a certain period for modification and amplification. The term "modification and amplification" means increasing the number of stem cells, and/or enhancing their function.

The method for in vitro amplifying and culturing the mononuclear cell fraction is not particularly limited, and any known method can be used. In one embodiment, stem cells are cultured in a serum-free medium or a serum medium containing one or two or more factors selected from the group consisting of a stem cell factor, interleukin-6, an FMS-like tyrosine kinase 3 ligand, thrombopoietin, and a vascular endothelial growth factor. Non-limitingly, the stem cells are cultured in a serum-free medium or a serum medium containing one or more, two or more, three or more, four or more, or five or more factors selected from the group consisting of a stem cell factor, interleukin-6, an FMS-like tyrosine kinase 3 ligand, thrombopoietin, and a vascular endothelial growth factor.

In one embodiment, the stem cells are cultured in a serum-free medium or a serum medium containing one or two or more factors selected from the group consisting of an FMS-like tyrosine kinase 3 ligand, thrombopoietin, and a vascular endothelial growth factor.

For example, PTL 2 describes a group of cells obtained by culturing mononuclear cells derived from bone marrow, umbilical cord blood, or peripheral blood in a serum-free medium containing a stem cell factor, interleukin-6, an FMS-like tyrosine kinase 3 ligand, thrombopoietin, and a vascular endothelial growth factor. PTL 4 describes a group of cells obtained by culturing mononuclear cells derived from bone marrow, umbilical cord blood, or peripheral blood in a medium containing serum and four or less factors selected from the group consisting of a stem cell factor, interleukin-6, an FMS-like tyrosine kinase 3 ligand, thrombopoietin, and a vascular endothelial growth factor. The in vitro amplified and cultured mononuclear cell fraction preferably has a characteristic of being rich in endothelial progenitor cells, and anti-inflammatory cells or immunotolerance-inducing cells. In one embodiment, it also includes anti-inflammatory M2 macrophages, T-lymphocyte subsets, and regulatory T-cells. The in vitro amplified and cultured mononuclear cell fraction preferably has a vascularization effect, an anti-fibrosis effect and/or an anti-inflammatory effect.

The method for in vitro amplifying and culturing the mononuclear cell fraction is not particularly limited, and any known method can be used. For example, NPLs 19 and 27 disclose specific methods for in vitro amplifying and culturing endothelial progenitor cells. For example, a medium supplemented with a VEGF, an SCF, an Flt-3 ligand, TPO, IL-6, and 1% antibiotics can be used. In the Examples in the present specification, culturing was performed for one week in a StemSpan medium supplemented with 50 ng/ml of VEGF, 100 ng/ml of SCF, 100 ng/ml of Flt-3 ligand, 20 ng/ml of TPO, 20 ng/ml of IL-6, and 1% antibiotics.

Alternatively, the method described in WO2006/090882 may be used. This method includes incubating endothelial progenitor cells in a serum-free medium containing one or two or more factors selected from the group consisting of a stem cell growth factor, interleukin-6, FMS-like tyrosine kinase 3, and thrombopoietin.

The term "mesenchymal stem cell" refers to somatic stem cells derived from mesodermal tissue (mesenchyme), which have potency to differentiate into cells belonging to the mesenchymal cell. Depending on the tissue from which the mesenchymal stem cells are collected, they are called adipose-derived mesenchymal stem cells (ASCs), bone marrow-derived mesenchymal stem cells, and the like. Mesenchymal stem cells are included in stromal cells. For example, when induced for differentiation, bone marrow stromal cells become cells belonging to the mesenchymal system (bone cells, cardiac muscle cells, chondrocytes, tenocytes, adipocytes, and the like).

In one embodiment, the mesenchymal stem cell is an adipose-derived mesenchymal stem cell, a bone marrow-derived mesenchymal stem cell, a peripheral blood-derived mesenchymal stem cell, or an umbilical cord blood-derived mesenchymal stem cell.

The "mesenchymal stem cell" may be either obtained from an organism or prepared artificially. A composition containing an effective amount of mesenchymal stem cell can also be used in the co-culturing. In one embodiment, the composition containing the mesenchymal stem cell is a stromal vascular cell fraction (SVF) (also referred to as "group of stromal vascular cells"). The stromal vascular cell fraction is a group of cells obtained by treating adipose tissue with collagenase. The stromal vascular cell fraction is a generally heterogeneous group of cells, and includes a group of cells derived from adipose tissue, in addition to blood-derived cells. The cells derived from adipose tissue include adipose stromal cells, which include the adipose-derived mesenchymal stem cells (ASCs).

The composition containing the in vitro amplified and cultured mononuclear cell fraction is used in a method for producing a composition for a treatment containing a mesenchymal stem cell and not containing a mononuclear cell fraction, the method including:

co-culturing a mesenchymal stem cell with the in vitro amplified and cultured mononuclear cell fraction; and separating the in vitro amplified and cultured mononuclear cell fraction prior to the treatment.

The composition is characterized by the co-culturing with a mesenchymal stem cell and the separation from the mesenchymal stem cell prior to the treatment with the mesenchymal stem cell. In other words, the in vitro amplified and cultured mononuclear cell fraction is co-cultured with a mesenchymal stem cell, but is separated from the mesenchymal stem cell when the treatment with the mesenchymal stem cell is performed. The in vitro amplified and cultured mononuclear cell fraction has been removed from the composition used in the treatment with the mesenchymal stem cell by the separation, and the composition contains the co-cultured mesenchymal stem cell alone. The composition containing the in vitro amplified and cultured mononuclear cell fraction is used only for the co-culturing with a mesenchymal stem cell, and is not used in the subsequent treatment with the mesenchymal stem cell.

The number of days for the co-culturing of the in vitro amplified and cultured mononuclear cell fraction with a mesenchymal stem cell is not particularly limited. In one embodiment, the in vitro amplified and cultured mononuclear cell fraction is co-cultured with a mesenchymal stem cell for a period in the range of from a half day (12 hours) to seven days. The terms "non-limitingly" and "in one embodiment" can be used herein synonymously. In one embodiment, they are co-cultured for a period in the range of from a half day to six days, from a half day to five days, from a half day to four days, from a half day to three days, from one to seven days, from one to six days, from one to five days, from one to four days, or from one to three days.

The ratios of cells between the in vitro amplified and cultured mononuclear cell fraction and mesenchymal stem cells to be co-cultured are not particularly limited. In one embodiment, mesenchymal stem cells are co-cultured with in vitro amplified and cultured mononuclear cells so that the number of mononuclear cells is 0.01 times or more, 0.03 times or more, 0.05 times or more, 0.07 times or more, 0.08 times or more, 0.09 times or more, or 0.1 times or more the number of mesenchymal stem cells. In one embodiment, the mesenchymal stem cells are co-cultured with the in vitro amplified and cultured mononuclear cells so that the number of mononuclear cells is ten times or less, eight times or less, five times or less, four times or less, three times or less, or two times or less the number of mesenchymal stem cells. Taking the amount of blood to be collected into consideration, it is preferable to keep the number of in vitro amplified and cultured mononuclear cells used low. In one embodiment, the ratio of the number of mesenchymal stem cells to the number of in vitro amplified and cultured mononuclear cells during the co-culturing is in the range of from 1:0.01 to 1:10. Preferably, it is in the range of from 1:0.05 to 1:4, or from 1:0.1 to 1:2.

The culture medium for the co-culturing is not particularly limited. In one embodiment, a medium suitable for culturing mesenchymal stem cells is used. The medium suitable for culturing mesenchymal stem cells can be appropriately prepared depending on the type of the mesenchymal stem cell. In one embodiment, as a culture medium for adipose-derived mesenchymal stem cells (ASCs), an ASC medium (for example, DMEM (high glucose) + 10% FBS + 1% penicillin/streptomycin) can be used, for example. Alternatively, a differentiation-inducing medium suitable for inducing differentiation of the mesenchymal stem cells into somatic cells can also be used. For example, an adipogenic differentiation inducing medium which induces differentiation of adipose-derived mesenchymal stem cells into adipocytes (for example, DMEM + 10%FBS + insulin +) may be used.

Alternatively, a medium suitable for culturing the in vitro amplified and cultured mononuclear cells may be used. As a culture medium for the in vitro amplified and cultured mononuclear cells, for example, IMDM + 10% FBS can be used.

The method for separating the composition containing the in vitro amplified and cultured mononuclear cell fraction, after being co-cultured with a mesenchymal stem cell, from the mesenchymal stem cell prior to the treatment with the mesenchymal stem cell is not particularly limited. For example, during the co-culturing, an insert may be used in which the mesenchymal stem cells and the in vitro amplified and cultured mononuclear cell fraction are separated by a membrane through which the cells cannot pass but the culture can pass. By culturing only the in vitro amplified and cultured mononuclear cell fraction in the insert and removing the insert after the co-culturing, the composition containing the in vitro amplified and cultured mononuclear cell fraction can be separated from the mesenchymal stem cells. As such a membrane, for example, a polyethylene terephthalate membrane with the pore size of 0.4 $\mu$m and the pore density of 2.0 $\times$ $10^6$ pores/cm$^2$ can be used.

Alternatively, the separation may be performed without using the insert, by directly co-culturing the in vitro amplified and cultured mononuclear cell fraction with mesenchymal stem cells and then sorting only the mesenchymal stem cells using a flow cytometer.

The composition containing the in vitro amplified and cultured mononuclear cell fraction increases the effect of the treatment with the mesenchymal stem cell by the action of the in vitro amplified and cultured mononuclear cell fraction.

The "treatment with the mesenchymal stem cell" is not particularly limited, as long as it is for a disease or a condition for which the treatment with the mesenchymal stem cell is effective. For example, mesenchymal stem cells may be used in a wide variety of treatments, such as application to regenerative therapy including reconstruction of bones, blood vessels, and cardiac muscles, and the like.

In one embodiment, the treatment with the mesenchymal stem cell is a treatment in which the effect thereof is enhanced by a vascularization effect, an anti-inflammatory effect, an anti-fibrosis effect and the like by the in vitro amplified and cultured mononuclear cell fraction.

Herein, "vascularization" is a concept including all forms of formation of blood vessels, such as vascular regeneration, and angiogenesis. The term "vascular regeneration" non-limitingly means the formation of blood vessels by stem cells. The term "angiogenesis" means the formation of blood vessels by the growth of existing blood vessels.

In one embodiment, the treatment with the mesenchymal stem cell is selected from the group consisting of tissue grafting, tissue regeneration, a treatment of an ischemic disease, a treatment of a neurodegenerative disease, a treatment of a heart disease, a treatment of a malignant tumor, a treatment of an inflammatory disease, and a treatment of an immune disease. In one embodiment, the treatment with the mesenchymal stem cell is selected from the group consisting of tissue regeneration, a treatment of an ischemic disease, a treatment of a neurodegenerative disease, a treatment of a heart disease, a treatment of a malignant tumor, a treatment of an inflammatory disease, and a treatment of an immune disease. In one embodiment, the treatment with the mesenchymal stem cell is selected from the group consisting of a treatment of an ischemic disease, a treatment of a neurodegenerative disease, a treatment of a heart disease, a treatment of a malignant tumor, a treatment of an inflammatory disease, and a treatment of an immune disease. In one embodiment, the treatment with the mesenchymal stem cell is a treatment of an ischemic disease.

The type of tissue in "tissue grafting, tissue regeneration" is not particularly limited, as long as it is biological tissue which can be grafted or regenerated. Examples of such tissue include fat, bone marrow, heart, kidney, and the like. Such a treatment is performed for the purpose of fat grafting for breast reconstruction after breast cancer surgery etc., bone marrow grafting (for treating leukemia, lymphoma, myeloma, and the like), or the like.

The tissue regeneration also include, for example, a treatment of a skeletal muscle injury, a treatment of a skin injury, tissue regeneration after prostate cancer surgery, a treatment of degenerative arthritis, a treatment of lumbar disc deformation, a treatment of a peripheral nerve injury, and the like. The tissue regeneration also include muscle regeneration (including muscle fiber regeneration).

The term "ischemic disease" refers to diseases caused by a decrease in a blood volume, which reduces a blood flow in tissue, resulting in a histological damage, such as cellular degeneration, atrophy, and fibrosis. Ischemia is broadly classified into occlusive ischemia, compressive ischemia, convulsive ischemia, and compensatory ischemia, depending on the cause. If ischemia continues, cellular degeneration, atrophy, and fibrosis occur. "Ischemic diseases" include limb ischemia, ischemic ulcer, ischemic heart diseases, brain infarction, and the like. The term "brain infarction" (or encephalomalacia) refers to a condition in which arteries supplying nutrients to the brain become obstructed or constricted to cause brain ischemia, which makes the brain tissue necrotic or nearly necrotic due to lack of oxygen or nutrients. It is classified as cerebral thrombosis or cerebral embolism. The term "limb ischemia" refers to a disease in which the arteries supplying blood to the legs become constricted or obstructed. When this arteriosclerosis obliterans increases in severity, it can lead to severe limb ischemia, causing symptoms such as pain and intractable ulcers. In the worst case scenario, it may require amputation of a lower limb.

The term "neurodegenerative disease" refers to diseases in which a certain group of nerve cells in the central nervous system gradually die, and are a type of cranial nerve disease. They include amyotrophic lateral sclerosis (ALS), Parkinson's disease, Alzheimer's disease, progressive supranuclear palsy, Huntington's disease, multiple system atrophy, spinocerebellar degeneration, a spinal cord injury, and the like. "Spinal cord injury" is a pathological condition in which the spine is damaged due to a strong external force applied mainly to the spinal column, resulting in a damage to the spinal cord. At the present time, it has been said that the spinal cord injury is difficult to be cured radically, but application of regenerative therapy using stem cells has been considered.

The term "heart disease" collectively refers to diseases of heart, and includes cardiac failure, endocarditis, a heart valve disease, pericarditis, a congenital heart disease, and other diseases (such as myocarditis and myocardial infarction).

The term "inflammatory disease" collectively refers to diseases which cause an abnormal condition, such as histological damages, due to some factors, resulting in a symptom. The inflammatory disease includes Crohn's disease, hepatic cirrhosis, hepatitis, ulcerative colitis, an inflammatory bowel disease, and the like.

The term "immune disease" (also referred to as "autoimmune disease") collectively refers to diseases caused by a breakdown in immunotolerance, in which the immune system responsible for recognizing and eliminating foreign substances overreacts and attacks the host's own normal cells and tissues. Autoimmune diseases can be classified into two types: systemic autoimmune diseases that affect the entire body; and organ-specific diseases that affect only specific organs. For example, connective tissue diseases such as rheumatoid arthritis and systemic lupus erythematosus (SLE) are systemic autoimmune diseases. Further, immune diseases also include a graft versus host disease (GVHD), multiple sclerosis, and the like.

In one embodiment, the treatment with the mesenchymal stem cell is fat grafting, bone marrow grafting, a treatment of a skeletal muscle injury, a treatment of a skin injury, tissue regeneration after prostate cancer surgery, a treatment of degenerative arthritis, a treatment of lumbar disc deformation, a treatment of a peripheral nerve injury, a treatment of limb ischemia, a treatment of ischemic ulcer, a treatment of an ischemic heart disease, a treatment of brain infarction, a treatment of amyotrophic lateral sclerosis (ALS), a treatment of Parkinson's disease, a treatment of Alzheimer's disease, a treatment of progressive supranuclear palsy, a treatment of Huntington's disease, a treatment of multiple system atrophy, a treatment of spinocerebellar degeneration, a treatment of a spinal cord injury, a treatment of cardiac failure, a treatment of endocarditis, a treatment of a heart valve disease, a treatment of pericarditis, a treatment of a congenital heart disease, a treatment of myocarditis, a treatment of myocardial infarction, a treatment of Crohn's disease, a treatment of hepatic cirrhosis, a treatment of hepatitis, a treatment of ulcerative colitis, a treatment of an inflammatory bowel disease, a treatment of a connective tissue disease, a treatment of a graft versus host disease (GVHD), or a treatment of multiple sclerosis.

In one embodiment, the treatment with the mesenchymal stem cell may be for any one or more of diseases selected from those described above.

The composition containing the in vitro amplified and cultured mononuclear cell fraction is used in a method for producing a composition for a treatment containing a mesenchymal stem cell and not containing a mononuclear cell fraction. The composition containing the in vitro amplified and cultured mononuclear cell fraction is a composition for increasing the effect of the treatment with the mesenchymal stem cell. It is used in the co-culturing with a mesenchymal stem cell containing the in vitro amplified and cultured mononuclear cell fraction, and is not used in the subsequent treatment with the mesenchymal stem cell.

The matters described in other sections are similarly applied to this section, unless particular contradiction occurs.

2. Composition Containing Mesenchymal Stem Cell

The present invention also provides a composition for a treatment containing a mesenchymal stem cell and not containing a mononuclear cell fraction.

In one embodiment, the composition is produced by:

    co-culturing a mesenchymal stem cell with an in vitro amplified and cultured mononuclear cell fraction; and
    separating the in vitro amplified and cultured mononuclear cell fraction prior to the treatment.

The composition is characterized by the co-culturing with the in vitro amplified and cultured mononuclear cell fraction and the separation from the in vitro amplified and cultured mononuclear cell fraction prior to the treatment.

Definitions of the terms "in vitro amplified and cultured," "mononuclear cell fraction," "mesenchymal stem cell," "co-culturing," "separate (separated)" and the like are as described in "1. Composition for Increasing Effect of Treatment with Mesenchymal Stem Cell." The matters described in other sections are similarly applied to this section, unless particular contradiction occurs.

The composition containing a mesenchymal stem cell is co-cultured with the in vitro amplified and cultured mononuclear cell fraction, and is then separated from the in vitro amplified and cultured mononuclear cell fraction prior to the treatment. In other words, the composition used in the treatment with the mesenchymal stem cell contains the mesenchymal stem cell alone co-cultured with the in vitro amplified and cultured mononuclear cell fraction, and the in vitro amplified and cultured mononuclear cell fraction has been removed from the composition by the separation. The composition containing the mesenchymal stem cell does not contain the in vitro amplified and cultured mononuclear cell fraction.

In one embodiment, the in vitro amplified and cultured mononuclear cell fraction is co-cultured with a mesenchymal stem cell for a half day to seven days.

In one embodiment, the mononuclear cell fraction is a mononuclear cell fraction derived from bone marrow, peripheral blood, or umbilical cord blood.

In one embodiment, the in vitro amplified and cultured mononuclear cell fraction is a group of cells rich in endothelial progenitor cells or anti-inflammatory cells or immunotolerance-inducing cells.

In one embodiment, the mesenchymal stem cell is an adipose-derived mesenchymal stem cell, a bone marrow-derived mesenchymal stem cell, a peripheral blood-derived mesenchymal stem cell, or an umbilical cord blood-derived mesenchymal stem cell.

The composition containing the mesenchymal stem cell is applied in an appropriate manner depending on the treatment to a subject, such as a human. In one embodiment, the composition containing the mesenchymal stem cell is preferably injected to a site of an organism to be treated with the mesenchymal stem cell (for example, by intravenous administration, intra-arterial administration, intramuscular administration, subcutaneous administration, intracerebroventricular administration, or application to tissue).

The subject of the treatment with the mesenchymal stem cell include any subject in need of the treatment with the mesenchymal stem cell. Non-limiting examples thereof include mammals, such as a human, non-human primates, a bovine, an equine, a porcine, an ovine, a caprine, a canine, a feline, and rodents (a mouse, a rat, and the like). Preferably, the subject is a human.

The composition containing the mesenchymal stem cell can be applied (used) to the subject by a medical professional (such as a doctor and a veterinarian) in an appropriate dosage and administration, depending on the treatment site, the severity of the disease/condition, and the conditions of the subject (age, body weight, height, medical history, physical condition, and the like). For example, when the composition is administered to a human subject, in one embodiment, the total number of cells of the in vitro amplified and cultured mononuclear cell fraction contained in the composition is in the range of from $1 \times 10^5$ to $5 \times 10^{11}$. The composition may be administered one time or a plurality of times.

3. Effect of Composition Containing In vitro amplified and cultured Mononuclear Cell Fraction

The composition containing the in vitro amplified and cultured mononuclear cell fraction according to the present invention increases the effect of the treatment with the mesenchymal stem cell. The term "increases an effect" means that the effect of the treatment with the mesenchymal stem cell is increased when a mesenchymal stem cell co-cultured with the composition containing the in vitro amplified and cultured mononuclear cell fraction is applied, as compared to the case in which the mesenchymal stem cell alone is applied to the subject.

In one embodiment, for example, when the treatment with the mesenchymal stem cell is grafting, and achieves at least one of the following effects to:

(i) increase a survival rate of grafted tissue;
(ii) increase an engrafting rate of a graft;
(iii) suppress fibrosis of a grafted tissue fragment;
(iv) improve a blood flow in the grafted tissue; or
(v) reduce inflammation due to the grafting.

Preferably, two, three, four, or five (all) of the above effects are achieved.

In one embodiment, the treatment with the mesenchymal stem cell is fat grafting, and achieves at least one of the following effects to:

(i) increase a survival rate of grafted adipose tissue;
(ii) increase an engrafting rate of a fat graft;

(iii) suppress fibrosis of a grafted adipose tissue fragment;
(iv) improve a blood flow in the grafted adipose tissue; or
(v) reduce inflammation due to the grafting.

Preferably, two, three, four, or five (all) of the above effects are achieved.

In one embodiment, the effect of the treatment with the mesenchymal stem cell, for example, at least one of the above effects (i) to (v), increases 1.1 times or more, 1.2 times or more, 1.3 times or more, 1.5 times or more, 1.8 times or more, or twice or more, when a mesenchymal stem cell co-cultured with the composition containing the in vitro amplified and cultured mononuclear cell fraction is applied, as compared to the case in which the mesenchymal stem cell alone is applied to the subject.

In one embodiment, the treatment with the mesenchymal stem cell is a treatment of ischemia, and achieves at least one of the following effects to:

(vi) improve a blood flow;
(vii) improve a function; or
(viii) improve a histological damage.

Preferably, two or three (all) of the above effects are achieved.

In one embodiment, the treatment with the mesenchymal stem cell is a treatment of limb ischemia, and achieves at least one of the following effects to:

(vi) improve a blood flow in lower limbs;
(vii) improve a function; or
(viii) improve a histological damage, such as ischemic ulcer.

Preferably, two or three (all) of the above effects are achieved.

The matters described in other sections are similarly applied to this section, unless particular contradiction occurs.

4. Method for Increasing Effect of Treatment with Mesenchymal Stem Cell

The present invention also provides a method for increasing an effect of a treatment with a mesenchymal stem cell.

In one embodiment, the method of the present invention includes:

co-culturing an in vitro amplified and cultured mononuclear cell fraction with a mesenchymal stem cell; and
separating the mesenchymal stem cell from the in vitro amplified and cultured mononuclear cell fraction.

Definitions of the terms "in vitro amplified and cultured," "mononuclear cell fraction," "mesenchymal stem cell," "co-culturing," "separate (separated)" and the like are as described in "1. Composition for Increasing Effect of Treatment with Mesenchymal Stem Cell."

The method for increasing an effect of a treatment with a mesenchymal stem cell according to the present invention further includes performing the treatment using the separated mesenchymal stem cell. Definitions of the terms "treatment with a mesenchymal stem cell" and the like are as described in "1. Composition for Increasing Effect of Treatment with Mesenchymal Stem Cell."

The matters described in other sections are similarly applied to this section, unless particular contradiction occurs.

5. Producing Method

The present invention also provides a method for producing a composition for a treatment containing a mesenchymal stem cell and not containing a mononuclear cell fraction.

In one embodiment, the producing method includes:

co-culturing a mesenchymal stem cell with an in vitro amplified and cultured mononuclear cell fraction; and
separating the in vitro amplified and cultured mononuclear cell fraction prior to the treatment.

Definitions of the terms "in vitro amplified and cultured," "mononuclear cell fraction," "mesenchymal stem cell," "co-culturing," "separate (separated)" and the like are as described in "1. Composition for Increasing Effect of Treatment with Mesenchymal Stem Cell." The matters described in other sections are similarly applied to this section, unless particular contradiction occurs.

In one embodiment, the in vitro amplified and cultured mononuclear cell fraction is co-cultured with a mesenchymal stem cell for a half day to seven days.

In one embodiment, the mononuclear cell fraction is a mononuclear cell fraction derived from bone marrow, peripheral blood, or umbilical cord blood.

In one embodiment, the in vitro amplified and cultured mononuclear cell fraction is a group of cells rich in endothelial progenitor cells or anti-inflammatory cells or immunotolerance-inducing cells.

In one embodiment, the mesenchymal stem cell is an adipose-derived mesenchymal stem cell, a bone marrow-derived mesenchymal stem cell, a peripheral blood-derived mesenchymal stem cell, or an umbilical cord blood-derived mesenchymal stem cell.

EXAMPLES

Hereinafter, the present invention will be described in detail with reference to Examples, but the present invention is not limited to the Examples. Modifications and variations can be easily added to the present invention by those skilled in the art based on the description herein, and are encompassed within the technical scope of the present invention.

Materials and Methods

(1) Preparation of Adipose-derived Mesenchymal Stem Cells (ASCs)

Human adipose tissue used was abdominal adipose tissue disposed of during a surgery in clinical studies approved by the Ethics Committee of Juntendo University.

After washing with PBS, the adipose tissue was digested with an equal amount of 0.1% collagenase 1 solution (WAKO PURE CHEMICAL). An equal amount of ASC medium (DMEM (high glucose, Invitrogen) + 10% FBS + 1% penicillin/streptomycin) was added to quench the enzymatic reaction. After centrifugal separation, the pellets were filtrated using a 100 $\mu$m cell strainer (manufactured by Corning). The residue was washed with the ASC medium, and then disseminated and cultured in a flask. The fifth passage ASCs were subjected to flow cytometry (BD LSRFortessa, BD Biosciences) to measure the expression of surface markers on the cells. Cells confirmed for being positive for CD73 and CD90 and negative for CD31, CD34, CD45, and HLA-DR, which is the characteristic of ASCs, were used in the Examples.

(2) Preparation of RE01 Cells

(i) Isolation of Mononuclear Cells

Using a BD Vacutainer (registered trademark) CPT (trademark) (manufactured by BD) blood collection tube for mononuclear cell separation, peripheral blood was collected from healthy human volunteers. After collecting the blood, the blood collection tube was centrifuged as it was, then transported, and stored under refrigeration after the arrival and until culturing was started. The part above the gel barrier in the CPT (trademark) blood collection tube containing mononuclear cells and plasma was collected into a centrifugation tube, and the inside of the blood collection tube, including above the gel barrier, was washed with a small amount of EDTA-PBS, and the cells were collected into the same centrifugation tube. The centrifugation tube into which the cells had been collected was filled up to the gauge line with EDTA-PBS, and then was subjected to centrifugal separation (300 $\times$ g, room temperature, 10 minutes) to collect the cells as sediment. The collected cells were incubated for five minutes at room temperature in an ACK hemolysis buffer (15 mL/tube) (Gibco, manufactured by Thermo-Fisher) in order to remove contaminating red blood cells. The composition of the ACK hemolysis buffer is as follows: 8,290 mg/l of $NH_4Cl$; 1,000 mg/l of $KHCO_3$; and 37 mg/l of $EDTA.Na_2 \cdot 2H_2O$. The cells were then diluted with EDTA-PBS up to the gauge line and were subjected to centrifugal separation ((200 $\times$ g, room temperature, 10 minutes) or (100 $\times$ g, room temperature, 15 minutes)) to collect the cells. This procedure was repeated two times.

(ii) Culture in Serum Medium

The collected mononuclear cells were suspended in a medium containing an IMDM (including GlutaMAX, Gibco) supplemented with 50 ng/mL of VEGF, 100 ng/mL of FLT-3 ligand, 20 ng/mL of TPO, 0.5% FBS, 0.5% HSA (Sekijuji Albumin 25%, Japan Blood Products Organization), 100 units/mL of penicillin, and 100 $\mu$g/mL of streptomycin. A portion of the suspension was used for cell counting using trypan blue. In the resulting cell proliferation medium, the cells were prepared to have a cell concentration of 1 $\times$ 10$^6$/mL, disseminated in a T25 flask, and were cultured for five days under normal culture conditions (37°C, 5% $CO_2$).

After the culturing for five days was finished, the culture medium was collected in a centrifugation tube, and centrifugal washing was performed three times for seven to ten minutes at 250 × g (about 1000 rpm). After the centrifugation, the cells were suspended in PBS. The resulting group of cells may be herein referred to as "RE-01."

Example 1. Co-culturing of ASCs with RE01 Cells

The ASCs prepared as in the section (1) were disseminated in a 6-well culturing plate at $7 \times 10^4$ cells/well, and cultured in an ASC medium (DMEM (high glucose, manufactured by Invitrogen) + 10% FBS + 1% penicillin/streptomycin) for one night.

An insert (manufactured by Corning) was then set, and the RE01 cells prepared as in the section (2) above were disseminated in the insert so that the number of cells was $3.5 \times 10^4$ cells/insert (1:0.5) or $0.7 \times 10^4$ cells/insert (1:0.1) for a vascularization assay, and also disseminated at $1.4 \times 10^5$ cells/insert (1:2) for an mRNA analysis.

The insert and the culture plate are separated by a membrane with a pore size of 0.4 $\mu$m, and the cells cannot pass through the membrane while the culture can pass through the membrane. In a state in which the insert with the disseminated RE01 cells had been set in the culture plate with the disseminated ASCs, culturing was performed in an ASC medium for three days at 37°C and under 5% $CO_2$.

Example 2. Tube Formation Assay (1)

Human umbilical vein endothelial cells (HUVECs) were purchased from LONZA, and cultured in an EGM-2 MV medium (manufactured by LONZA) according to the manufacturer's instructions. The tenth passage cells were cultured for one hour in a nutrient-deprived medium (EBM-2) (manufactured by LONZA) supplemented with no growth factor or FBS, and then detached with 0.05% trypsin-EDTA and collected.

From the co-cultured product (after three days of co-culturing) of the ASCs and the RE01 cells prepared in the Example 1, the insert (RE01 cells) was removed, and only the ASCs in the 6-well culturing plate were separated. The ASCs were washed with PBS, and then detached with 0.05% trypsin-EDTA and collected. In 200 $\mu$L of PBS, HUVEC 20000 cells and ASC 4500 cells were mixed, and were disseminated in a 24-well culturing plate coated with 200 $\mu$L/well of Matrigel Matrix (Corning Inc.). The cells were then cultured for six hours at 37°C and under 5% $CO_2$. The cells were photographed under a microscope (Olympus IX83) every one hour, and the number of closed circles were counted.

As ASCs, ASC 4500 cells were co-cultured with RE01 cells, at ratios of 1:0.5 and 1:0.1, respectively, for three days, and then the RE01 cells were removed. Groups with thus prepared ASCs, that is, ASC (+ RE01 1:0.5) 4500 group and ASC (+ RE01 1:0.1) 4500 group, were used (ASC (+ RE01 1:0.5) 4500 **(A)** and ASC (+ RE01 1:0.1) 4500 **(▼)** in Fig. 3). As controls, a group in which only the HUVEC 20000 cells had been added (HUVEC20000 (●) in Fig. 3) and a group using ASC 4500 cells not co-cultured with the RE01 cells (ASC (single) 4500 (■) in Fig. 3) were used. For each group, the number of closed circles was counted.

The results are illustrated in Fig. 3. In the ASC (+ RE01 1:0.5) 4500 group and the ASC (+ RE01 1:0.1) 4500 group, the number of closed circles after three hours significantly increased approximately 1.3 times, as compared to the group using the ASCs not co-cultured with the RE01 cells (ASC (single)) (Fig. 3B). It demonstrated that the vascularization capability of the ASCs was enhanced by co-culturing with the RE01 cells (co-culturing for three days) regardless of the number of RE01 cells.

Example 3. mRNA Expression Analysis (1)

After co-culturing the ASCs and the RE01 cells for three days in the Example 1, the ASCs were separated, RNAs were purified, and cDNAs were synthesized. In this Example, the resulting cDNAs were subjected to real-time PCR for measuring and analyzing expression levels of respective genes, that is, ANGPT-2, IGF-1, ADAMTS-1, and IL-1β.

After the co-culturing in the Example 1, the ASCs were separated and washed with PBS, and then the Total RNAs were extracted using RNeasy Micro Kit (Qiagen). From 1 to 2 $\mu$g of RNAs, cDNAs were synthesized using High-Capacity RNA-to-cDNA Kit (Applied Biosystems). Then reagents were adjusted using TaqMan fast advanced master mix (Applied Biosystems), and measurement and analysis were performed using StepOne Plus (Applied Biosystems). All of the TaqMan probes used for the analysis were purchased from Applied Biosystems

Angiopoietin ANGPT-2 (Hs00169867_m1);
Insulin-like growth factor (IGF)-1 (Hs00153126_m1);
Extracellular matrix degrading enzyme ADAMTS-1 (Hs00199608_m1);
IL-1β (Hs01555410_m1); and
18S-rRNA (Hs03928990_g1).

Using the ΔΔCt method (comparative Ct method), the mRNA expressions were analyzed. In the ΔΔCt method, comparison is performed by the following three calculations:

$$\Delta Ct = (target\ gene\ Ct) - (internal\ control\ Ct);$$

$$\Delta\Delta Ct = (each\ sample\ \Delta Ct) - (reference\ sample\ average\ \Delta Ct^0);$$

and plugging the obtained ΔΔCt into 2-(-ΔΔCt) and comparing the numbers.

As the internal control Ct, the value of 18S-rRNA was used.

As illustrated in Fig. 4A, the ASCs co-cultured with the RE01 cells for three days had increased mRNA expression levels of ANGPT-2, IGF-1, and IL-1β, demonstrating that the angiogenesis capability was enhanced. Furthermore, as illustrated in Fig. 4B, the ASCs co-cultured with less RE01 cells (+ RE01 1:0.5, 1:0.1) for three days had increased mRNA expression levels of IGF-1, IL-1β, and ADAMTS-1, demonstrating that the co-culturing with the RE01 cells enhanced the angiogenesis capability of the ASCs regardless of the number of RE01 cells.

Example 4. Tube FormationAssay (2)

The test was conducted as in the tube formation assay (1) in the Example 2, but the ASCs were co-cultured with the RE01 cells for one day, instead of three days.

From the co-cultured product (after one day of co-culturing) of the ASCs and the RE01 cells prepared in the Example 1, the insert (RE01 cells) was removed, and only the ASCs in the 6-well culturing plate were separated. The ASCs were washed with PBS, and then detached with 0.05% trypsin-EDTA and collected. In 200 μL of PBS, HUVEC 20000 cells and ASC 4500 cells were mixed, and were disseminated in a 24-well culturing plate coated with 200 μL/well of Matrigel Matrix (Corning Inc.). The cells were then cultured for six hours at 37°C and under 5% $CO_2$. The cells were photographed under a microscope (Olympus IX83) every one hour, and the number of closed circles was counted.

As ASCs, ASC 4500 cells were co-cultured with RE01 cells, at ratios of 1:0.5 and 1:0.1, respectively, for one day, and then the RE01 cells were removed. Groups with thus prepared ASCs, that is, ASC (+ RE01 1:0.5) 4500 group and ASC (+ RE01 1:0.1) 4500 group, were used (ASC (+ RE01 1:0.5) 4500 **(A)** and ASC (+ RE01 1:0.1) 4500 **(▼)** in Fig. 5). As controls, a group in which only HUVEC 20000 cells had been added (HUVEC 20000 (●) in Fig. 5) and a group using ASC 4500 cells not co-cultured with the RE01 cells (ASC (single) 4500 (■) in Fig. 5) were used. For each group, the number of closed circles was counted.

The results are illustrated in Fig. 5. In the ASC (+ RE01 1:0.5) 4500 group and the ASC (+ RE01 1:0.1) 4500 group, the number of closed circles after three hours significantly increased approximately 1.5 times, as compared to the group using the ASCs not co-cultured with the RE01 cells (ASC (single)) (Fig. 5B). It demonstrated that the vascularization capability of the ASCs was enhanced by co-culturing with the RE01 cells (co-culturing for one day) regardless of the number of RE01 cells.

Example 5. mRNA Expression Analysis (2)

The test was conducted as in the mRNA expression analysis (1) in the Example 3, but the ASCs were co-cultured with the RE01 cells for one day, instead of three days.

After co-culturing the ASCs and the RE01 cells for one day in the Example 1, the ASCs were separated, RNAs were purified, and cDNAs were synthesized. In this Example, the resulting cDNAs were subjected to real-time PCR for measuring and analyzing expression levels of respective genes, that is, IGF-1, IL-1β, and ADAMTS-1.

After the co-culturing in the Example 1, the ASCs were separated and washed with PBS, and then the Total RNAs were extracted using RNeasy Micro Kit (Qiagen). From 1 to 2 μg of RNAs, cDNAs were synthesized using High-Capacity RNA-to-cDNA Kit (Applied Biosystems). Then reagents were adjusted using TaqMan fast advanced master mix (Applied Biosystems), and measurement and analysis were performed using StepOne Plus (Applied Biosystems). All of the TaqMan probes used for the analysis were purchased from Applied Biosystems.

Insulin-like growth factor (IGF)-1 (Hs00153126_m1);

IL-1β (Hs01555410_m1);
Extracellular matrix degrading enzyme ADAMTS-1 (Hs00199608 m1); and
18S-rRNA (Hs03928990_g1).

Using the ΔΔCt method (comparative Ct method), the mRNA expressions were analyzed. In the ΔΔCt method, comparison is performed by the following three calculations:

$$\Delta Ct = (\text{target gene Ct}) - (\text{internal control Ct});$$

$$\Delta\Delta Ct = (\text{each sample } \Delta Ct) - (\text{reference sample average } \Delta Ct^0);$$

and

plugging the obtained $\Delta\Delta Ct$ into $2^{-(\Delta\Delta Ct)}$ and comparing the numbers.

As the internal control Ct, the value of 18S-rRNA was used.

As illustrated in Fig. 6, the ASCs co-cultured with the RE01 cells for one day had increased mRNA expression levels of IGF-1 and IL-1$\beta$, demonstrating that the angiogenesis capability of the ASCs was enhanced by the co-culturing with the RE01 cells regardless of the number of RE01 cells.

Example 6. Experiment Using Nude Mouse Model Grafted with Fat to Back

ASCs were isolated from adipose tissue (of abdomen or thigh) disposed of during a surgery. They were cultured in an ASC medium (DMEM (high glucose, manufactured by Invitrogen) + 10% FBS + 1% penicillin/streptomycin), and the ASC of the fourth to sixth passages were used.

Mononuclear cells were isolated from peripheral blood from healthy human volunteers. The isolated mononuclear cells were suspension-cultured for five days in a medium containing an IMDM (including GlutaMAX, Gibco) supplemented with 50 ng/mL of VEGF, 100 ng/mL of FLT-3 ligand, 20 ng/mL of TPO, 0.5% FBS, 0.5% HSA (Sekijuji Albumin 25%, Japan Blood Products Organization), 100 units/mL of penicillin, and 100 $\mu$g/mL of streptomycin (RE01 growth medium), thereby producing RE01 cells.

The ASCs were disseminated in a 6-well plate at $7 \times 10^4$ cells/well, and cultured in an ASC medium for one night. RE01 cells were then disseminated in an insert at $0.7 \times 10^4$ cells/well. The cells were co-cultured in an ASC medium for three days at 37°C and under 5% $CO_2$, and the ASC were collected.

Female BALB/c nu-nu mice of eight weeks old were used as nude mice. In a 1 mL syringe, 200 $\mu$L of human adipose tissue and $2 \times 10^4$ ASC cells/20 $\mu$L of PBS were placed and mixed. 200 $\mu$L of fat was injected to the mice at each of two positions on the back using a branded needle of 18G. The wound was sutured with one stitch of 5-0 nylon thread. The grafting was performed under isoflurane inhalation anesthesia. As controls, a fat-only group and a conventional ASC-mixed group without the co-culturing were used.

CT scans (Latheta LCT-200, Hitachi, Ltd) were taken every one week, and the grafted tissue was excised four weeks after the grafting. The excised grafts were cut in half and fixed in 10% neutral buffered formalin, and then paraffin-embedded sections were prepared. The sections were immunostained using an anti-CD31 antibody (ab28364, abcam), an anti-Perilipin antibody (GP29, Progen), and an anti-iNOS antibody (NB300-605, Novusbio).

Fig. 7 illustrates photographs of the grafted adipose tissue fragments excised after four weeks. The volume was measured based on the CT-scanned images using ImageJ. The volume on Day 0, immediately after the grafting, was set as 100%, and the volume retention rate was calculated (Fig. 8). The ASC-mixed group with the ASCs co-cultured with the RE01 cells maintained the volume of the grafted adipose tissue, as compared to the fat-only group and the ASC-mixed group with the conventional, not co-cultured ASCs.

The immunostained specimens were photographed using Keyence BZ-X 710 and analyzed using ImageJ. Fig. 9 illustrates the results of histological analysis of the fat-grafted model mice four weeks after the grafting. The quality of fat was evaluated by calculating the proportion of the area of Perilipin antibody-positive adipocytes relative to the total area of the specimen (Fig. 9, left). The number of microvessels was evaluated by measuring the number of CD31-positive blood vessels in the entire specimen and calculating the number of blood vessels per 1 mm$^2$ (Fig. 9, center). Infiltration of inflammatory cells was evaluated by counting the number of iNOS antibody-positive inflammatory macrophages in the entire specimen (Fig. 9, right).

It revealed that the ASC-mixed group with the ASCs co-cultured with the RE01 cells had a higher quality of fat, larger number of blood vessels, and less inflammation, as compared to the fat-only group and the ASC-mixed group with the conventional, not co-cultured ASCs.

**Claims**

1.   A composition for a treatment comprising a mesenchymal stem cell and not comprising a mononuclear cell fraction, wherein the composition is produced by:

co-culturing a mesenchymal stem cell with an in vitro amplified and cultured mononuclear cell fraction; and separating the in vitro amplified and cultured mononuclear cell fraction prior to the treatment.

2. A composition comprising an in vitro amplified and cultured mononuclear cell fraction for use in a method for producing a composition for a treatment comprising a mesenchymal stem cell and not comprising a mononuclear cell fraction, the method comprising:

co-culturing a mesenchymal stem cell with the in vitro amplified and cultured mononuclear cell fraction; and separating the in vitro amplified and cultured mononuclear cell fraction prior to the treatment.

3. The composition according to claim 1 or 2, wherein the in vitro amplified and cultured mononuclear cell fraction is co-cultured with a mesenchymal stem cell for a half day to seven days.

4. The composition according to any one of claims 1 to 3, wherein the mononuclear cell fraction is a mononuclear cell fraction derived from bone marrow, peripheral blood, or umbilical cord blood.

5. The composition according to any one of claims 1 to 4, wherein the in vitro amplified and cultured mononuclear cell fraction is a group of cells rich in endothelial progenitor cells or anti-inflammatory cells or immunotolerance-inducing cells.

6. The composition according to any one of claims 1 to 5, wherein the mesenchymal stem cell is an adipose-derived mesenchymal stem cell, a bone marrow-derived mesenchymal stem cell, a peripheral blood-derived mesenchymal stem cell, or an umbilical cord blood-derived mesenchymal stem cell.

7. The composition according to any one of claims 1 to 6, wherein the treatment with the mesenchymal stem cell is a treatment in which the effect thereof is enhanced by a vascularization effect, an anti-inflammatory effect, an anti-fibrosis effect and the like by the in vitro amplified and cultured mononuclear cell fraction.

8. The composition according to any one of claims 1 to 7, wherein the treatment with the mesenchymal stem cell is selected from the group consisting of tissue grafting, tissue regeneration, a treatment of an ischemic disease, a treatment of a neurodegenerative disease, a treatment of a heart disease, a treatment of a malignant tumor, a treatment of an inflammatory disease, and a treatment of an immune disease.

9. The composition according to any one of claims 1 to 8, wherein the treatment with the mesenchymal stem cell is selected from the group consisting of fat grafting, bone marrow grafting, a treatment of a skeletal muscle injury, a treatment of a skin injury, tissue regeneration after prostate cancer surgery, a treatment of degenerative arthritis, a treatment of lumbar disc deformation, a treatment of a peripheral nerve injury, a treatment of limb ischemia, a treatment of ischemic ulcer, a treatment of an ischemic heart disease, a treatment of brain infarction, a treatment of amyotrophic lateral sclerosis (ALS), a treatment of Parkinson's disease, a treatment of Alzheimer's disease, a treatment of progressive supranuclear palsy, a treatment of Huntington's disease, a treatment of multiple system atrophy, a treatment of spinocerebellar degeneration, a treatment of a spinal cord injury, a treatment of cardiac failure, a treatment of endocarditis, a treatment of a heart valve disease, a treatment of pericarditis, a treatment of a congenital heart disease, a treatment of myocarditis, a treatment of myocardial infarction, a treatment of Crohn's disease, a treatment of hepatic cirrhosis, a treatment of hepatitis, a treatment of ulcerative colitis, a treatment of an inflammatory bowel disease, a treatment of a connective tissue disease, a treatment of a graft versus host disease (GVHD), and a treatment of multiple sclerosis.

10. The composition according to claim 9, wherein the treatment with the mesenchymal stem cell is grafting, and achieves at least one of the following effects to:

(i) increase a survival rate of grafted tissue;
(ii) increase an engrafting rate of a graft;
(iii) suppress fibrosis of a grafted tissue fragment;
(iv) improve a blood flow in the grafted tissue; or
(v) reduce inflammation due to the grafting.

11. The composition according to claim 9, wherein the treatment with the mesenchymal stem cell is a treatment of ischemia, and achieves at least one of the following effects to:

(vi) improve a blood flow;
(vii) improve a function; or
(viii) improve a histological damage.

12. A method for increasing an effect of a treatment with a mesenchymal stem cell, the method comprising:

co-culturing an in vitro amplified and cultured mononuclear cell fraction with a mesenchymal stem cell; and separating the mesenchymal stem cell from the in vitro amplified and cultured mononuclear cell fraction.

13. The method according to claim 12, further comprising performing the treatment using the separated mesenchymal stem cell.

14. A method for producing a composition for a treatment comprising a mesenchymal stem cell and not comprising a mononuclear cell fraction, the method comprising:

co-culturing a mesenchymal stem cell with an in vitro amplified and cultured mononuclear cell fraction; and separating the in vitro amplified and cultured mononuclear cell fraction prior to the treatment.

# Fig. 1

Fig. 2

IN VITRO
AMPLIFIED AND
CULTURED
MONONUCLEAR
CELL FRACTION

AFTER CO-
CULTURING
FOR 3 DAYS

HUVEC
ASC

ASC

MATRIGEL

MONONUCLEAR CELL FRACTION IS REMOVED TO
USE ONLY ASC IN TUBE FORMATION ASSAY

Fig. 3

A

HUVEC 20000      ASC (+RE01 1:0.5) 4500

ASC (single) 4500      ASC (+RE01 1:0.1) 4500

B

ASC (single) 4500

ASC (+RE01 1:0.5) 4500

ASC (+RE01 1:0.1) 4500

* p<0.05
n=6, Turkey's multiple comparisons test

EP 4 635 504 A1

Fig. 4

# Fig. 5

A

B

* p<0.05
n=4, Turkey's multiple comparisons test

EP 4 635 504 A1

Fig. 6

*p<0.05, **p<0.01
n=5, Turkey's multiple comparisons test

Fig. 7

FAT GRAFT AFTER 4 WEEKS

Fat only    +ASC    +CO-CULTURED ASC

Fig. 8

Fig. 9

HISTOLOGICAL ANALYSIS OF FAT
GRAFT AFTER 4 WEEKS

☐ Fat only

▨ +ASC

■ +CO-CULTURED ASC

PROPORTION OF PERIPIN-POSITIVE
LIVING ADIPOCYTES

NUMBER OF CD31-POSITIVE
MICROVESSELS

INFILTRATION OF
INFLAMMATORY CELLS

QUALITY OF FAT

NUMBER OF BLOOD VESSELS

**Perilipin**

**CD31**

**iNOS**

* p<0.05, ** p<0.01

## INTERNATIONAL SEARCH REPORT

| International application No. |
| --- |
| **PCT/JP2023/044423** |

### A. CLASSIFICATION OF SUBJECT MATTER

*A61K 35/15*(2015.01)i; *A61K 35/14*(2015.01)i; *A61K 35/28*(2015.01)i; *A61K 35/51*(2015.01)i; *A61P 1/04*(2006.01)i; *A61P 1/16*(2006.01)i; *A61P 9/00*(2006.01)i; *A61P 9/04*(2006.01)i; *A61P 9/10*(2006.01)i; *A61P 17/02*(2006.01)i; *A61P 19/02*(2006.01)i; *A61P 25/00*(2006.01)i; *A61P 25/14*(2006.01)i; *A61P 25/16*(2006.01)i; *A61P 25/28*(2006.01)i; *A61P 29/00*(2006.01)i; *A61P 35/00*(2006.01)i; *A61P 37/02*(2006.01)i; *A61P 37/06*(2006.01)i; *A61P 43/00*(2006.01)i; *C12N 5/0775*(2010.01)i; *C12N 5/078*(2010.01)i

FI: A61K35/15; A61K35/14; A61K35/28; A61K35/51; A61P1/04; A61P1/16; A61P9/00; A61P9/04; A61P9/10; A61P17/02; A61P19/02; A61P25/00; A61P25/14; A61P25/16; A61P25/28; A61P29/00; A61P35/00; A61P37/02; A61P37/06; A61P43/00 121; C12N5/0775; C12N5/078

According to International Patent Classification (IPC) or to both national classification and IPC

### B. FIELDS SEARCHED

Minimum documentation searched (classification system followed by classification symbols)

A61K35/15; A61K35/14; A61K35/28; A61K35/51; A61P1/04; A61P1/16; A61P9/00; A61P9/04; A61P9/10; A61P17/02; A61P19/02; A61P25/00; A61P25/14; A61P25/16; A61P25/28; A61P29/00; A61P35/00; A61P37/02; A61P37/06; A61P43/00; C12N5/0775; C12N5/078

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
| --- | --- | --- |
| X | US 2019/0002832 A1 (ASSOCIATION INSTITUT DE MYOLOGIE) 03 January 2019 (2019-01-03) claims 16, 17, 19-21, paragraphs [0075]-[0077], [0084]-[0091], [0098]-[0100], [0114] | 1-8, 10, 12-14 |
| Y | | 7-10 |
| A | | 11 |
| Y | BROWN, C, et al., Mesenchymal stem cells: Cell therapy and regeneration potential, J Tissue Eng Regen Med., 2019, vol. 13, pp. 1738-1755 table 2, fig. 4 | 7-10 |

☑ Further documents are listed in the continuation of Box C.   ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
| --- | --- |
| **20 February 2024** | **05 March 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT

International application No.

**PCT/JP2023/044423**

### C. DOCUMENTS CONSIDERED TO BE RELEVANT

| Category* | Citation of document, with indication, where appropriate, of the relevant passages | Relevant to claim No. |
|---|---|---|
| Y | SAEEDI, P, et al., A revealing review of mesenchymal stem cells therapy, clinical perspectives and Modification strategies, Stem Cell Investig, 2019, vol. 6:34, pp. 1-18, http://dx.doi.org/10.21037/sci.2019.08.11<br>  fig. 1, 2, table 2 | 7-10 |
| Y | GUADIX, JA, et al., Characteristics, applications and prospects of mesenchymal stem cells in cell therapy, Med Clin (Barc)., 2017, vol. 148, no. 9, pp. 408-414<br>  tables 2, 3 | 7-10 |
| P, X | 古川聖美ほか. 生体外増幅単核球RE01と脂肪由来間葉系幹細胞を用いた新規脂肪移植法の開発. 第32回日本形成外科学会基礎学術集会プログラム・抄録集. October 2023, p. 134, 09-2, non-official translation (FURUKAWA, Satomi et al. Development of Novel Fat Graft Method Using In Vitro Amplification Mononuclear RE01 and Fat-Derived Mesenchymal Stem Cells. Program and Abstracts of the 32nd Research Council Meeting of the Japan Society for Plastic and Reconstructive Surgery.)<br>  entire text | 1-14 |

Form PCT/ISA/210 (second sheet) (July 2022)

**INTERNATIONAL SEARCH REPORT**
Information on patent family members

International application No.

**PCT/JP2023/044423**

| Patent document cited in search report | Publication date (day/month/year) | Patent family member(s) | Publication date (day/month/year) |
|---|---|---|---|
| US 2019/0002832 A1 | 03 January 2019 | WO 2017/109204 A1<br>EP 3393236 A1 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019146131 A
- WO 2006090882 A
- WO 20140561154 A
- WO 2021131261 A

**Non-patent literature cited in the description**

- *Plast Reconstr Surg.*, February 2013, vol. 131 (2), 185-191
- *Langenbecks Arch Klin Chir Ver Dtsch Z Chir.*, 1893, vol. 22, 66-69
- *Otolaryngol Head Neck Surg.*, April 1990, vol. 102 (4), 314-321
- *Ann Plast Surg.*, July 2005, vol. 55 (1), 63-68
- *Exp Biol Med (Maywood).*, November 2005, vol. 230 (10), 742-748
- *Dermatol Surg.*, December 2006, vol. 32 (12), 1437-1443
- *Plast Reconstr Surg Glob Open.*, 07 October 2013, vol. 1 (6), e40
- *Arch Plast Surg.*, March 2015, vol. 42 (2), 150-158
- *Plast Reconstr Surg.*, May 2012, vol. 129 (5), 1081-1092
- *Plast Reconstr Surg.*, June 2015, vol. 135 (6), 1607-1617
- *Science*, 14 February 1997, vol. 275 (5302), 964-967
- *Circulation.*, 06 February 2001, vol. 103 (5), 634-637
- *Nat Med.*, April 2001, vol. 7 (4), 430-436
- *Circ Res.*, 06 July 2001, vol. 89 (1), E1-7
- *J Am Coll Cardiol.*, 17 December 2003, vol. 42 (12), 2073-2080
- *FASEB J.*, June 2005, vol. 19 (8), 992-994
- *Plast Reconstr Surg.*, June 2008, vol. 121 (6), 1929-1942
- *Diabetes.*, August 2010, vol. 59 (8), 1974-1983
- *Stem Cells Transl Med.*, February 2012, vol. 1 (2), 160-171
- *Mol Biol Cell.*, December 2002, vol. 13 (12), 4279-4295
- *J Plast Reconstr Aesthet Surg.*, November 2013, vol. 66 (11), 1494-1503
- *Aesthet Surg J.*, 01 July 2017, vol. 37 (3), 46-58
- *Stem Cells.*, January 2009, vol. 27 (1), 266-274
- *Circulation.*, 10 February 2004, vol. 109 (5), 656-663
- *PLoS One.*, 2012, vol. 7 (9), e45621
- *Lancet.*, 28 September 2013, vol. 382 (9898), 1113-1120
- *Diabetes.*, September 2013, vol. 62 (9), 3207-3217
- *Circ Res.*, 24 June 2011, vol. 109 (1), 20-37
- *Stem Cell Res Ther.*, 28 February 2013, vol. 4 (1), 20
- *J Am Heart Assoc.*, 25 June 2014, vol. 3 (3), e000743
- *Japanese Journal of Thrombosis and Hemostasis*, 2014, vol. 25 (5), 593-602